Europäisches Patentamt

European Patent Office

Office européen des brevets

(19)

(11) Veröffentlichungsnummer: **0 259 312**
**B1**

(12) **EUROPÄISCHE PATENTSCHRIFT**

(45) Veröffentlichungstag der Patentschrift: **16.08.90**

(51) Int. Cl.⁵: **A 61 B 17/14**

(21) Anmeldenummer: **86902768.0**

(22) Anmeldetag: **06.05.86**

(86) Internationale Anmeldenummer:
**PCT/AT86/00041**

(87) Internationale Veröffentlichungsnummer:
**WO 86/06609 20.11.86 Gazette 86/25**

(54) **VORRICHTUNG ZUR OSTEOTOMIE.**

(30) Priorität: **07.05.85 AT 1368/85**

(43) Veröffentlichungstag der Anmeldung:
**16.03.88 Patentblatt 88/11**

(45) Bekanntmachung des Hinweises auf die
Patenterteilung:
**16.08.90 Patentblatt 90/33**

(84) Benannte Vertragsstaaten:
**BE DE FR GB IT NL**

(56) Entgegenhaltungen:
**FR-A-2 304 322**
**US-A-4 509 511**

(73) Patentinhaber: **Ender, Hans Georg, Dr.**
**Krenngasse 3**
**A-1180 Wien (AT)**

(72) Erfinder: **Ender, Hans Georg, Dr.**
**Krenngasse 3**
**A-1180 Wien (AT)**

(74) Vertreter: **Brauneiss, Leo et al**
**Patentanwälte Dipl.-Ing. Peter Boeckmann,**
**Dipl.-Ing. Leo Brauneiss Strohgasse 10**
**A-1030 Wien (AT)**

**Beschreibung**

Die Erfindung betrifft eine Vorrichtung zur Osteotomie, mit einem in einer Ebene senkrecht zur Vorschubrichtung bogenförmig, insbesondere kreisbogenförmig, gekrümmten Sägeblatt, das über ein Distanzstück von einer oszillierenden Antriebseinrichtung zu einer hin- und hergehenden Bewegung der Sägekante angetrieben ist, wobei das Distanzstück einen in Vorschubrichtung des Sägeblattes verlaufenden Führungskanal aufweist, in dem im Knochen verankerbare Führungsmittel eingesetzt sind.

Es sind bereits verschiedene Sägen zur Durchführung der Osteotomie bekannt, nämlich Sägen mit einem geraden Sägeblatt, Sägen mit einem Sägeblatt mit gekrümmter Sägekante und auch Sägen, bei welchen die Sägekante einen geschlossenen Kreis bildet. Während die beiden erstegenannten Sägeblätter hauptsächlich für die vollständige Durchtrennung eines Knochens, beispielsweise bei einer Amputation, herangezogen werden, dient des zuletzt erwähnte Sägeblatt hauptsächlich für die Entnahme eines Knochenstückes zu Untersuchungszwecken.

Die Sägen werden entweder händisch oder mittels einer in der Regel pneumatisch angetriebenen antriebseinrichtung bewegt. Hiebei ist es bekannt, dem Sägeblatt ein drehende Bewegung oder eine oszillierende Bewegung zu erteilen.

Die erfindungsgemäße Vorrichtung ist in der Hauptsache gedacht für eine Knochenbehandlung in der Folge eines schlecht verheilten Knochenbruches, wenn nämlich die Knochenfagmente an der Bruchstelle nicht die richtige Lage zueinander einnehmen, sondern beispielsweise an Stelle der gestreckten Lage einen Winkel miteinander einschließen. In diesem Falle ist er erforderlich, den Knochen im Bereich der Bruchstelle zu durchtrennen, die dadurch entstehenden beiden Knochenteile in die gegenseitige richtige Lage einzurichten, so daß eine neuerliche Heilung der Bruchstelle in dieser richtigen Lage erfolgt. Heibei ist es wichtig, daß die beiden Knochenteile möglichst großflächig aneinander anliegen, denn die Heilung geht umso besser und problemloser vor sich, je größer die Berührungsfläche zwischen den beiden Knochenteilen ist.

Soll eine Korrektur bei einer verheilten Bruchstelle vorgenommen werden, bei welcher die beiden Knochenteile nicht die gewünschte gestreckte Lage einnehmen, sondern einen stumpfen Winkel miteinander einschließen, so ist man bisher so vorgegangen, im Bereich der verheilten Bruchstelle ein keilförmiges Knochenstück herauszuschneiden, wobei der Keil so geformt ist, daß eine Postionierung der beiden Knockenteile in der gestreckten Lage möglich ist. Diese bekannte Behandlungsmethode bringt jedoch zahlreiche Nachteile mit sich. So verursacht das Herausschneiden des Keiles eine Verringerung der Gesamtlänge des Knochens, was in der Regel auch mit funktionellen Nachteilen verbunden ist. Des weiteren ist es sehr schwierig, einerseits den erforderlichen Keilwinkel genau festzustellen und anderseits einen Keil mit diesem Keilwinkel herauszuschneiden. Es muß daher häufig nachgeschnitten werden, um eine gute Berührung der gerade gerichteten Knochenteile an der Schnittstelle sicherzustellen, wie dies für einen problemlosen Heilvorgang erforderlich ist. Durch das Nachschneiden erfolgt jedoch eine weitere Verkürzung des Knochens. Ein weiterer Nachteil ist, daß auch bei einem exakten Schnitt die Berührungsfläche zwischen den beiden Knochenteilen dem Knochenquerschnitt entspricht, also etwa kreisförmig ist und daher verhältnismäßig klein ist.

Es ist auch bekannt, die Berührungsstelle an der aufgetrennten Bruchstelle V-förmig auszubilden. Hiedurch wird zwar scheinbar die Berührungsfläche vergrößert, tatsächliche ergeben sich jedoch nur einzelne Berührungspunkte, vor allem deshalb, da der dachförmige Vorsprung des einen Knochenteiles bei der Einrichtung der Knochenteile in die gestreckte Lage nicht exakt in die V-förmige Ausnehmung des anderen Knockenteiles paßt. Dadurch wird in der Praxis die Fläche, an welcher die beiden Knochenteile tatsächlich aneinander anliegen, wesentlich reduziert. Außerdem ist es bei dieser Methode erforderlich, die V-förmige Vertiefung aus dem einen Knochenteil herauszumeißeln, was äußerst schwierig und zeitaufwendig ist und häufig ein Aussplittern des Knochens zur folge hat.

Es ist weiters die sogenannte hexagonale Osteotomie bekannt, bei welcher die Berührungsfläche die Form eines Teilumrisses eines Vielekkes aufweist, das aus dem Knochen herausgeschnitten wird. Eine solche Form der Berührungsfläche ist jedoch äußerst schwierig herstellbar und weist außerdem den Nachteil auf, daß die Lage der Knochenteile immer nur um einer Betrag relativ zueinander versetzt werden kann, der den Zentrumswinkel des Vieleckes entspricht, so daß eine genaue Einstellung der gewünschten Lage dieser Knochenteile nicht möglich ist.

Aus der US—A—4 509 511 ist eine Vorrichtung zur Osteotomie bekannt, mit welcher die Berührungsstelle bogenförmig ausgebildet werden kann. Diese Vorrichtung weist ein in einer Ebene senkrecht zur Vorschubrichtung kreisbogenförmig gekrümmtes Sägeblatt auf, das über ein Distanzstück zu einer hin- und hergehenden Bewegung der Sägekante angetrieben ist. Das Distanzstück weist einen in Vorschubrichtung des Sägeblattes verlaufenden Führungskanal auf, in dem ein im Knochen verankerter Führungsstift eingesetzt ist. Nachteilig ist bei diesen bekannten Anordnung, daß zu Beginn des Sägevorganges ein großer Teil des im Knochen verankerten Führungsstiftes nicht im Führungskanal geführt ist, wodurch die Exaktheit der Führung beeinträchtigt wird.

Die vorliegende Erfindung hat sich zur Aufgabe gestellt, eine Vorrichtung zur Osteotomie zu schaffen, bei welcher eine exakte Führung des Sägeblattes beim Vorschub und damit eine einwandfreie Schnittsführung auch bereits zu Beginn des Sägevorganges gewährleistet ist. Zur Lösung dieser Aufgabe schlägt die Erfindung vor, daß im Distanzstück eine Führungshülse für die Führungsmittel angeordnet ist, welche Führungshülse in der einen Endstellung aus dem Distanzstück herausragt und sich zumindest über einen Teil der Länge des Sägeblattes—in Vorschubrichtung gemessen—erstreckt, und welche im Distanzstück in Vorschubrichtung entgegen der Kraft einer Feder od.dgl. in die andere Endstellung verschiebbar ist, in welcher zumindest ein Teil ihrer Länge sich im Inneren des Distanzstückes befindet.

Vorzugsweise sind die Führungsmittel von einem in den Knochen eingebohrten Bohrdraht gebildet, der leicht mittels eines üblichen Bohrgerätes in den Knochen eingesetzt werden kann.

Das Sägeblatt kann erfindungsgemäß mit einem Trägerkörper verbunden sein, der mit einer Bohrung für den Führungskanal versehen ist und mit dem Distanzstück drehfest verbunden ist. Eine solche Anordnung ermöglicht eine leichte Austauschbarkeit der Sägeblätter, und zwar sowohl abgenützter Sägeblätter gegen neue Sägeblätter als auch Sägeblätter größerer Krümmung gegen Sägeblätter kleinerer Krümmung und umgekehrt. Vorzugsweise ist hiebei der Trägerkörper von einer sich im wesentlichen senkrecht zur Vorschubrichtung des Sägeblattes erstreckenden Platte gebildet, an deren Seitenwand das Sägeblatt mit dem der Sägekante in Vorschubrichtung gegenüberliegenden Ende befestigt ist. Die Befestigung kann hiebei lösbar sein.

Insbesondere bei Verwendung kleinerer Sägeblätter ist es jedoch, um einen raschen Ersatz der Sägeblätter zu ermöglichen, von Vorteil, wenn erfindungsgemäß das Sägeblatt mit dem Trägerkörper untrennbar verbunden ist und daher gemeinsam mit dem Trägerkörper ausgetauscht werden kann.

Um den Trägerkörper auf einfache Weise mit dem Distanzstück lösbar zu verbinden, wie dies zur Übertragung der hin- und hergehenden Bewegung von der Antriebseinrichtung über das Distanzstück auf den Trägerkörper und damit auf das Sägeblatt erforderlich ist, steht erfindungsgemäß vom Distanzstück ein den Führungskanal bzw. die Führungshülse umschließender Zapfen ab, der in der Bohrung des Trägerkörpers eingesetzt ist. Dieser Zapfen kann hiebei als Gewindezapfen ausgebildet und in eine Gewindebohrung im Trägerkörper eingeschraubt sein. Es ist aber auch möglich, den vom Distanzstück abstehenden Zapfen mit der Bohrung im Trägerkörper über einen Bajonettverschluß zu verbinden.

Fallweise kommt es vor, daß die Schnittstelle am Knochen zumindest teilweise von Bändern, Sehnen od.dgl. bedeckt ist, die durch den Schnitt nicht beschädigt werden dürfen. Um auch in diesem Fall den erforderlichen bogenförmigen Schnitt durchführen zu können, kann erfindungsgemäß der Trägerkörper über einen Steg mit dem die Sägekante aufweisenden Teil verbunden sein, welcher Teil von einem quer zur Längsrichtung des Steges verlaufenden Balken gebildet ist. Bei dieser Ausführungsform kann der die Sägekante aufweisende Balken bei der Durchführung des bogenförmigen Schnittes unterhalb der Bänder, Sehnen od.dgl. hindurchgeführt werden, ohne daß diese beschädigt werden.

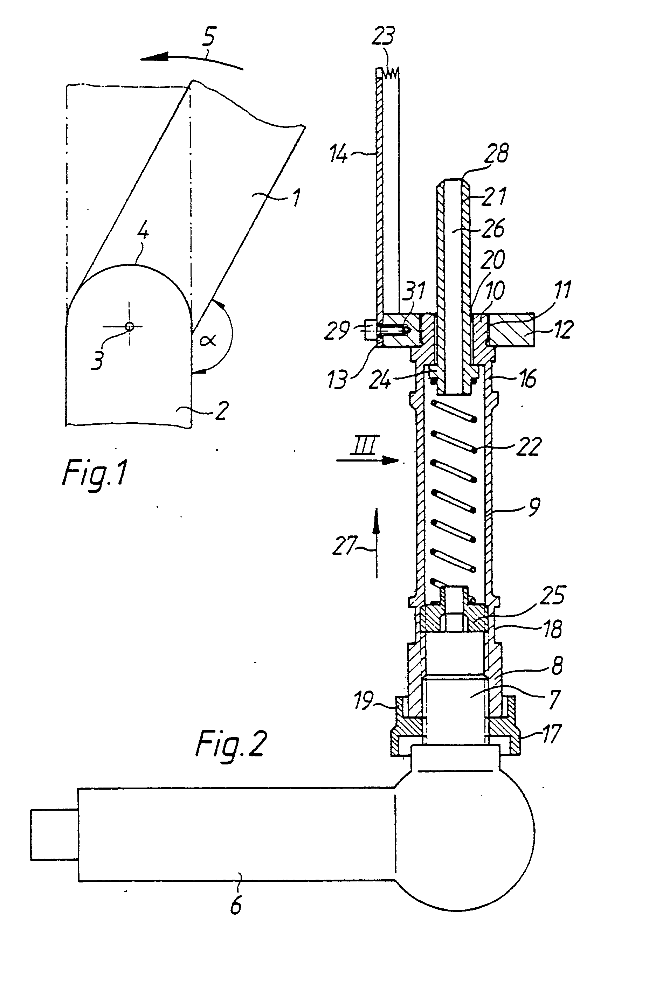

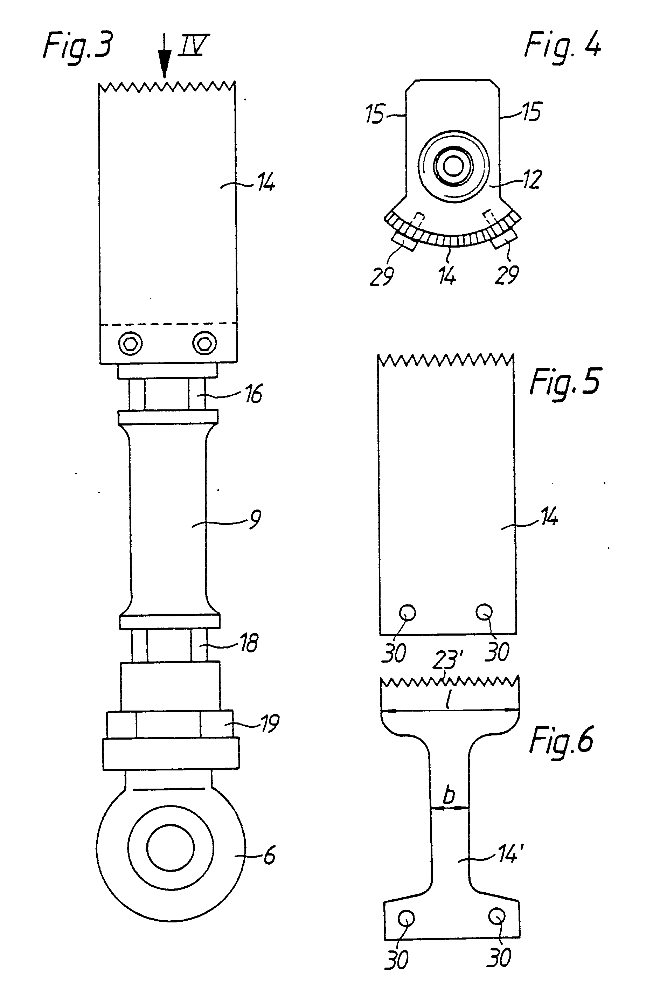

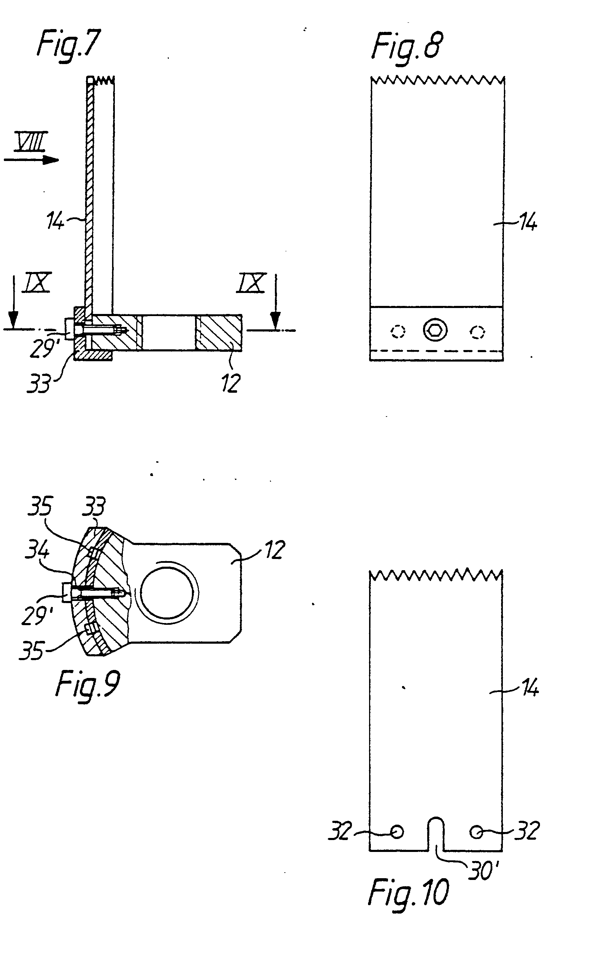

In der Zeichnung ist die Erfindung an Hand von Ausführungsbeispielen schematisch veranschaulicht. Fig. 1 zeigt in vereinfachter Darstellung zwei verheilte Knochenteile, die an der Bruchstelle in unerwünschter Weise einen stumpfen Winkel miteinander einschließen, sowie die Schnittführung mittels der erfindungsgemäßen Vorrichtung zur Osteotomie. Fig. 2 stellt einen Längsschnitt durch die erfindungsgemäße Vorrichtung dar. Fig. 3 zeigt eine Ansicht der erfindungsgemäßen Vorrichtung in Richtung des Pfeiles III in Fig. 2 und Fig. 4 stellt eine Ansicht in Richtung des Pfeiles IV in Fig. 3 dar, Fig. 5 stellt eine Ausführung eines Sägeblattes einer erfindungsgemäßen Vorrichtung dar und Fig. 6 zeigt eine abgewandelte Ausführungsform einen solchen Sägeblattes. Fig. 7 stellt im Schnitt eine Variante der Befestigung des Sägeblattes am Trägerkörper dar. Fig. 8 zeigt eine Ansicht in Richtung des Pfeiles VIII in Fig. 7 und Fig. 9 einen Schnitt nach der Linie IX—IX in Fig. 7. Fig. 10 zeigt das bei der Anordnung nach den Fig. 7 bis 9 verwendete Sägeblatte in ausgebautem Zustand.

Detaillierte Beschreibung der Zeichnungen

In Fig. 1 sind zwei Knochenteile 1, 2 dargestellt, die an der Bruchstelle in unerwünschter Weise derart verheilt sind, daß sie einen stumpfen Winkel α miteinander einschließen. Um eine Geradestellung zu bewirken, wird mittels der erfindungsgemäßen Vorrichtung zur Osteotomie die bereits verheilte Bruchstelle aufgetrennt. Hiebei wird zunächst an der Stelle 3 ein Bohrdraht, beispielsweise ein Steinman-pin, in den Knochen hineingebohrt, welcher als Führungsstift für die Vorschubbewegung des Sägeblattes dient, wie in der Folge bei der Beschreibung der erfindungsgemäßen Vorrichtung näher erläutert werden wird. Mit diesem Sägeblatt wird der Knochen durchtrennt, wobei die Schnittführung bei 4 ersichtlich ist. Der Knochenteil 1 kann hierauf in Richtung des Pfeiles 5 verschwenkt werden, bis er die gewünschte, in Fig. 1 strichpunktiert eingezeichnete Lage einnimmt, in welcher er während des neuerlichen Heilungsprozesses fixiert wird, Wie aus der Zeichnung ersichtlich ist, kann die Verschwenkung kontinuierlich vorgenommen werden, und es erfolgt keine Verkürzung der Knochenlänge.

Die erfindungsgemäße Vorrichtung weist ein mit einer bekannten, vorzugsweise pneumatischen Antriebseinrichtung kuppelbares Getriebe 6 auf, dessen mit einem Gewinde versehener Antriebszapfen 7 eine oszillierende Bewegung vollführt. Auf den Antriebszapfen 7 ist das mit einem Gewinde versehene Ende 8 eines Distanzstückes 9 aufgeschraubt. Am gegenüberliegenden Ende weist das Distanzstück 9 einen Gewindezapfen 10 auf, auf den eine Gewindebohrung 11 eines plattenförmigen Trägekörpers 12 aufgeschraubt ist, auf dessen Seitenwand 13 ein bogenförmig gekrümmtes Sägeblatt 14 befestigt ist. Eine solche Anordnung ermöglicht eine leichte Austauschbarkeit sowohl der Sägeblätter 14, und zwar sowohl abgenützter Sägeblätter gegen neue Sägeblätter als auch Sägeblätter größerer Krümmung gegen Sägeblätter kleinerer Krümmung bzw. umgekehrt.

Insbesondere bei Verwendung kleinerer Sägeblätter 14 ist es jedoch, um einen raschen Ersatz der Sägeblätter zu ermöglichen, von Vorteil, wenn erfindungsgemäß das Sägeblatt mit dem Trägerkörper 12 untrennbar verbunden ist und daher gemeinsam mit dem Trägerkörper 12 ausgetauscht wird, welche Ausführungsform in der Zeichnung nicht dargestellt ist. Um eine drehfeste Verbindung zwischen dem Distanzstück 9 und dem Trägerkörper 12 zu gewährleisten, ist es erforderlich, die Gewindebohrung 11 des Trägerkörpers 12 fest auf den Gewindezapfen 10 aufzuschrauben. Um dies zu ermöglichen, weist der Trägerkörper 12 Angriffsflächen 15 für einen Schraubenschlüssel auf und das Distanzstück 9 besitzt bei 16 einen Sechskantquerschnitt für einen Schraubenschlüssel, so daß eine Relativverdrehung zwischen dem Distanzstück 9 und dem Trägerkörper 12 mittels dieser beiden Schraubenschlüssel vorgenommen werden kann. Es ist aber auch möglich, daß der vom Distanzstück 9 abstehende Zapfen 7 mit der Bohrung 11 im Trägerkörper 12 über einen Bajonettverschluß verbunden ist.

Um das Distanzstück 9 einerseits drehfest mit dem Gewindezapfen 7 der Antriebseinrichtung 6 zu verbinden, anderseits die gewünschte Lage des Sägeblattes in Bezug auf die Antriebseinrichtung 6 einstellen zu können, ist auf dem Gewindezapfen 7 eine Gegenmutter 17 angeordnet, welche mit der Stirnseite des mit dem Gewinde versehenen Endes 8 des Distanzstückes 9 zusammenwirkt. Um eine relative Verdrehung zwischen dem Ende 8 und der Gegenmutter 17 mittels Schraubenschlüsseln zu ermöglichen, ist das Distanzstück 9 mit einem weiteren Sechskantteil 18 zum Ansetzen eines Schraubenschlüssels versehen und die Gegenmutter 17 ist im Bereich 19 gleichfalls als Sechskantmutter ausgebildet.

Im Gewindezapfen 10 des Distanzstückes 9 befindet sich eine Bohrung 20, in welcher eine Führungshülse 21 geführt ist. Diese Führungshülse ist durch eine Schraubenfeder 22, die sich im Inneren des hohl ausgebildeten Distanzstückes befindet, in Richtung gegen die Sägekante 23 des Sägeblattes 14 gedrückt, bis der Flansch 24 der Führungshülse 21 an der Stirnfläche des Hohlraumes des Distanzstückes 9 anliegt. Die Feder 22 ist auf einem Widerlager 25 abgestützt, das in das Gewinde des Endes 8 der Führungshülse 9 eingeschraubt ist. Der Kanal 26 der Führungshülse 21 nimmt den in den Knochen an der Stelle 3 (siehe Fig. 1) verankerten Führungsstift auf, so daß die erfindungsgemäße Vorrichtung beim Vorschub während des Sägevorganges in Richtung des Pfeiles 27 geführt ist, wobei die Sägekante 23 eine oszillierende Bewegung ausführt und hiedurch ein Schnitt entsprechend der Schnittlinie 4 in Fig. 1 bewirkt wird. Gelangt die Stirnfläche 28 der Führungshülse 21 am Knochen zur Anlage, so weicht beim weiteren Vorschub der erfindungsgemäßen Vorrichtung diese Führungshülse entgegen der Kraft der Feder 22 in den Hohlraum des Distanzstückes 9 aus. Es ist somit immer eine exakte Führung der erfindungsgemäßen Vorrichtung durch den in den Kanal 26 der Führungshülse 22 eingesetzten Führungsstift gewährleistet.

Wie insbesondere aus Fig. 4 hervorgeht, ist das Sägeblatt 14 in einer Ebene senkrecht zu seiner Vorschubrichtung bogenförmig gekrümmt ausgebildet, so daß die erforderliche Schnittführung gewährleistet ist.

Bei der Ausführungsform nach den Fig. 2 bis 5 erfolgt die Befestigung des Sägeblattes 14 an der Seitenwand 13 des Trägerkörpers 12 über zwei Schrauben 29, die durch Öffnungen 30 im Sägeblatt 14 hindurchgeführt sind und in Gewindebohrungen 31 im Trägerkörper 12 eingeschraubt sind. Eine solche Ausbildung ermöglicht einen Ersatz des Sägeblattes 14, wenn dieses abgenützt ist, ohne daß gleichzeitig auch der Trägerkörper 12 ausgewechselt werden muß. Bei kleineren Sägeblättern kann aber auch das Sägeblatt 14 untrennbar mit dem Trägerkörper 12 verbunden sein. Bei einem Ersatz eines Sägeblattes durch ein solches mit einem anderen Krümmungsradius wird zweckmäßig das Sägeblatte zusammen mit dem Trägerkörper 12 ausgewechselt.

Fig. 6 zeigt die Ausbildung eines Sägeblattes 14', bei welchem der Trägerkörper 12 über einen Steg mit dem die Sägekante 23' aufweisenden Teil verbunden sein kann, welcher Teil von einem quer zur Längsrichtung des Steges verlaufenden Balken gebildet ist, welcher somit eine henkerbeilähnliche Form besitzt. Wie aus dieser Fig. ersichtlich ist, ist die Länge 1 der Sägekante 23' wesentlich größer als die Breite b des zwischen der Sägekante 23' und dem mit dem Trägerkörper 12 verbundenen Ende des Sägeblattes befindlichen Steges. Eine solche Ausbildung des Sägeblattes 14' ist dann erforderlich, wenn die zu durchtrennende Knochenstelle von Bändern, Sehnen od.dgl. bedeckt ist, da dann die Sägekante unterhalb dieser Bänder eingesetzt werden kann und die gewünschte Schnittführung ohne Verletzung bzw. Beschädigung der Bänder vorgenommen werden kann.

Die Fig. 7 bis 10 zeigen eine weitere Ausführung der lösbaren Befestigung des Sägeblattes 14 am Trägerkörper 12. Wie aus Fig. 10 hervorgeht, weist das Sägeblatt 14 in der Mitte ein gegen den Rand des Sägeblattes offenes Langloch 30' auf. Daneben sind Öffnungen 32 vorgesehen. Die Befestigung des Sägeblattes 14 erfolgt unter Vermittlung einer im Querschnitt winkelförmigen Schiene 33, die mit einer mit dem Langloch 30' fluchtenden Bohrung 34 für eine in den Trägerkörper 12 eingeschraubte Schraube 29' ausgestattet ist. Vom Trägerkörper 12 stehen Zapfen 35 ab.

Um das Sägeblatt 14 an der Seitenwand 13 des Trägerkörpers 12 zu fixieren, wird die Schraube 29' so weit herausgeschraubt, daß die Schiene 33 derart von der Seitenwand 13 abhebt, daß eine Einführen des Sägeblattes 14 in den Zwischenraum zwischen der Seitenwand 13 des Trägerkörpers 12 und der Schiene 33 trotz der vorspringenden Zapfen 35 möglich ist. Der Schraubenschaft wird hiebei vom seitlich offenen Langloch 30' aufgenommen. Beim Anziehen der Schraube 29' wird das Sägeblatt 14 in Richtung zur Seitenwand 13 des Trägerkörpers 12 verschoben, wobei die Zapfen 35 in die Öffnungen 32 eindringen und eine zusätzliche Fixierung des Sägeblattes 14 bewirken.

## Patentansprüche

1. Vorrichtung zur Osteotomie, mit einem in einer Ebene senkrecht zur Vorschubrichtung (27) bogenförmig, insbesondere kreisbogenförmig, gekrümmten Sägeblatt (14, 14'), das über ein Distanzstück (9) von einer oszillierenden Antriebseinrichtung (6) zu einer hin- und hergehenden Bewegung der Sägekante (23) angetrieben ist, wobei das Distanzstück (9) einen in Vorschubrichtung (27) des Sägeblattes (14, 14') verlaufenden Führungskanal (26) aufweist, in dem im Knochen (2) verankerbare Führungsmittel eingesetzt sind, dadurch gekennzeichnet, daß im Distanzstück (9) eine Führungshülse (21) für die Führungsmittel angeordnet ist, welche Führungshülse (21) in der einen Endstellung aus dem Distanzstück (9) herausragt und sich zumindest über einen Teil der Länge des Sägeblattes (14)—in Vorschubrichtung (27) gemessen—erstreckt, und welche im Distanzstück (9) in Vorschubrichtung (27) entgegen der Kraft einer Feder (22) od.dgl. in die andere Endstellung verschiebbar ist, in welcher zumindest ein Teil ihrer Länge sich im Inneren des Distanzstückes (9) befindet.

2. Vorrichtung nach Anspruch 1, dadurch gekennzeichnet, daß die Führungsmittel von einem in den Knochen eingebohrten Bohrdraht gebildet sind.

3. Vorrichtung nach Anspruch 1, dadurch gekennzeichnet, daß das Sägeblatt (14) mit einem Trägerkörper (12) verbunden ist, der mit einer Bohrung (11) für den Führungskanal (26) versehen ist und mit dem Distanzstück (9) drehfest verbunden ist.

4. Vorrichtung nach Anspruch 3, dadurch gekennzeichnet, daß der Trägerkörper (12) von einer sich im wesentlichen senkrecht zur Vorschubrichtung (27) des Sägeblattes (14) erstreckenden Platte gebildet ist, an deren Seitenwand (13) das Sägeblatt (14) mit dem der Sägekante (23) in Vorschubrichtung gegen-überliegenden Ende befestigt ist.

5. Vorrichtung nach Anspruch 3, dadurch gekennzeichnet, daß das Sägeblatt (14) mit dem Trägerkörper (12) untrennbar verbunden ist.

6. Vorrichtung nach Anspruch 1, dadurch gekennzeichnet, daß vom Distanzstück (9) ein den Führungskanal (26) bzw. die Führungshülse (21) umschließender Zapfen (10) absteht, der in der Bohrung (11) des Trägerkörpers (12) eingesetzt ist.

7. Vorrichtung nach Anspruch 6, dadurch gekennzeichnet, daß der vom Distanzstück (9) abstehende Zapfen (10) als Gewindezapfen und die Bohrung (11) im Trägerkörper (12) als entsprechende Gewindebohrung ausgebildet sind.

8. Vorrichtung nach Anspruch 6, dadurch gekennzeichnet, daß der vom Distanzstück (9) abstehende Zapfen (10) mit der Bohrung (11) im Trägerkörper (12) über einen Bajonettverschluß verbunden ist.

9. Vorrichtung nach Anspruch 3, dadurch gekennzeichnet, daß der Trägerkörper (12) über einen Steg mit dem die Sägekante (23') aufweisenden Teil verbunden ist, welcher Teil von einem quer zur Längsrichtung des Steges verlaufenden Balken gebildet ist.

## Revendications

1. Dispositif pour l'ostéotomie, comprenant une lame de scie recourbée (14, 14') qui présente une forme en arc, et en particulier en arc de cercle, dans un plan perpendiculaire à la direction de l'avance (27) et qui est entraînée au moyen d'un dispositif d'entraînement oscillant (6) par l'intermédiaire d'une pièce intercalaire (9) pour impartir un mouvement de va-et-vient à l'arête (23) de la scie, la pièce intercalaire (9) présentant un canal de guidage (26) qui s'étend dans la direction (27) de l'avance de la lame de scie (14, 14') et dans lequel sont introduits des moyens de guidage pouvant être ancrés dans l'os (2), caractérisé par le fait qu'un manchon de guidage (21) destiné aux moyens de guidage est disposé dans la pièce intercalaire (9), lequel manchon de guidage (21), dans l'une des positions extrêmes, fait saillie hors de la pièce intercalaire (9) et s'étend sur une partie au moins de la longueur de la lame de scie (14)—mesurée dans la direction (27) de l'avance—cependant qu'il peut amené dans l'autre position extrême par coulissement dans la pièce intercalaire (9) dans la direction (27) de l'avance à l'encontre de la force d'un ressort (22) ou similaire, position dans laquelle une partie au moins de sa longueur se trouve à l'intérieur de la pièce intercalaire (9).

2. Dispositif selon la revendication 1, caractérisé par le fait que les moyens de guidage sont constitués par un fil de forage chirurgical enfoncé dans l'os par perçage.

3. Dispositif selon la revendication 1, caractérisé par le fait que la lame de scie (14) est reliée à un support (12) qui est muni d'un perçage (11) destiné au canal de guidage (26) et qui est relié à la pièce intercalaire (9) sans pouvoir tourner par rapport à elle.

4. Dispositif selon la revendication 3, caractérisé par le fait que le support (12) est constitué par une plaque qui s'étend pour l'essentiel perpendiculairement à la direction (27) de l'avance de la lame de scie (14), et à la paroi latérale (13) de laquelle la lame de scie (14) est fixée par son extrémité opposée à l'arête (23) de la scie dans la direction de l'avance.

5. Dispositif selon la revendication 3, caractérisé par le fait que la lame de scie (14) est reliée au support (12) de manière inséparable.

EP 0 259 312 B1

9 | 10

6. Dispsitif selon la revendication 1, caractérisé par le fait que la pièce intercalaire (9) se prolonge par une douille (10) qui entoure le canal de guidage (26) ou le manchon de guidage (21), respectivement, et qui est introduite dans le perçage (11) du support (12).

7. Dispositif selon la revendication 6, caractérisé par le fait que la douille (10) qui prolonge la pièce intercalaire (9) est réalisée sous la forme d'une douille filetée et que le perçage (11) ménagé dans le support (12) est réalisé sous la forme d'un perçage fileté correspondant.

8. Dispositif selon la revendication 6, caractérisé par le fait que la douille (10) qui prolonge la pièce intercalaire (9) est reliée au perçage (11) ménagé dans le support (12) par l'intermédiaire d'une fermeture à baïonnette.

9. Dispositif selon la revendication 3, caractérisé par le fait que le suport (12) est relié par une patte à la partie qui présente l'arête (23') de la scie et qui est formée par une barre s'étendant transversalement par rapport à la direction longitudinale de la patte.

**Claims**

1. A device for osteotomy, with a saw web (14, 14') which is curved in an arc-shaped manner, particularly in a circular-arc-shaped manner, in a plane perpendicular to the direction of advance (27) and is driven, via a distance piece (9), by an oscillating drive-device (6) so that a to-and-fro movement of the saw edge (23) is caused, the distance piece (9) having a guide channel (26) which runs in the direction of advance (27) of the saw web (14, 14') and in which guide means anchorable in the bone (2) are inserted, characterized in that a guide sleeve (21) for the guide means is arranged in the distance piece (9), which guide sleeve (21) in one final position projects from the distance piece (9) and extends over at least a part of the length of the saw web (14) (measured in the direction of advance (27)), and which guide sleeve is displaceable in the distance piece (9), in the direction of advance (27) and against the power of a spring (22) or the like, into the other final position in which at least a part of its length is located within the distance piece (9).

2. A device in accordance with Claim 1, characterized in that the guide means are formed by a bore wire bored into the bone.

3. A device in accordance with Claim 1, characterized in that the saw web (14) is connected to a carrier body (12) provided with a bore (11) for the guide channel (26) and secured to the distance piece (9) in a rotation-proof manner.

4. A device in accordance with Claim 3, characterized in that the carrier body (12) is formed from a plate which extends substantially perpendicular to the direction of advance (27) of the saw web (14) and on the side wall (13) of which the saw web (14) is secured to the end opposite the saw edge (23) in the direction of advance.

5. A device in accordance with Claim 3, characterized in that the saw web (14) is inseparably connected to the carrier body (12).

6. A device in accordance with Claim 1, characterized in that a peg (10) embracing the guide channel (26) and the guide sleeve (21) projects from the distance piece (9), this peg being inserted in the bore (11) of the carrier body (12).

7. A device in accordance with Claim 6, characterized in that the peg (10) projecting from the distance piece (9) is formed as a threaded peg and the bore (11) in the carrier body (12) is formed as a corresponding threaded bore.

8. A device in accordance with Claim 6, characterized in that the peg (10) projecting from the distance piece (9) is connected to the bore (11) in the carrier body (12) via a bayonet catch.

9. A device in accordance with Claim 3, characterized in that the carrier body (12) is connected via a bridge to the part with the saw edge (23'), this part being formed from a bar running at right angles to the longitudinal direction of the bridge.

*Fig.1*

*Fig.2*

*Fig.3*

IV

14

16

9

18

19

6

*Fig. 4*

15    15

12

29    14    29

*Fig.5*

14

30    23'    30

l

b

*Fig.6*

14'

30    30

Fig.7

VIII →

14

IX

IX

29'

33

12

Fig.8

14

Fig.9

35    33

34

29'

35

12

Fig.10

14

32    32

30'

3